# EUROPEAN PATENT SPECIFICATION

(11) **EP 0 812 833 B1**
(45) Date of publication and mention of the grant of the patent: **05.09.2001**
(21) Application number: 97109158.2
(22) Date of filing: 05.06.1997
(51) Int. Cl.: C07D 249/00, C07D 249/18, C08J 9/00, C09D 11/02, D06N 7/00

(54) **Water soluble triazole derivatives and their use as blowing inhibitor in chemical embossing processes**
Wasserlösliche Triazolderivate und ihre Verwendung als Treibmittelinhibitoren in chemischen Prägeverfahren
Dérivés de triazole hydrosolubles et leur utilisation comme inhibiteurs d'agents d'expansion dans des procédés de gaufrage chimique

(30) Priority: 10.06.1996 US 658511
(43) Date of publication of application: 17.12.1997
(73) Proprietor: Armstrong World Industries, Inc., Lancaster Pennsylvania 17603 (US)
(72) Inventor: Sideman, Carl E., Lititz, PA 17543 (US); Snyder, Donald, M., Saline, MI 48176 (US)
(74) Representative: von Füner, Alexander, Prof.h.c. Dr.

(56) References cited:
- WO-A-96/07670
- GB-A- 1 466 558
- GB-A- 1 511 593
- GB-A- 1 514 359
- US-A- 4 464 276
- US-A- 5 169 435
- US-A- 5 441 563

## Description

The invention relates to blowing agent inhibitors and their use. In particular, the invention is directed to water soluble triazole derivatives which are effective blowing agent inhibitors.

It is well known to those skilled in the art that foamed plastic surfaces may be textured by the process commonly referred to as "chemical embossing", wherein the surface of a foamable polymer composition is printed with an ink composition containing an agent which inhibits foaming in the printed areas when the surface is subsequently subjected to a heat treatment. The areas which have not been printed over thus expand normally on heating while expansion in the printed areas containing the inhibitor is retarded, resulting in a textured surface with depressions in those areas printed with the inhibited ink.

A wide range of compounds have been claimed to act as inhibitors for chemical embossing of floor and wall covering surfaces. Carboxylic acid anhydrides such as trimellitic anhydride (TMA) being among the most commonly used industrially. However, compounds such as TMA, while suitable for solvent-based printing inks, are hydrolytically unstable and thus are not readily usable in the aqueous ink formulations rapidly gaining in importance in large scale printing operations due to environmental concerns over VOC emission from solvent-based inks.

Triazole compounds such as benzotriazole (BTA) and tolyltriazole (TTA) are also widely used in solvent-based inks for chemical embossing. These compounds do not hydrolyze to inactive form on contact with water as do carboxylic acid anhydrides like TMA. However their use in aqueous ink systems is hindered by a lack of substantial water solubility.

US-5169435 discloses an aqueous, foam-retarding, printing ink composition consisting essentially of
(A) about 40% to about 80% by weight of an aqueous thermoplastic or thermosetting printing ink;
(B) about 1% to about 25% by weight of a foaming or blowing agent modifier or inhibitor selected from the group consisting of benzotriazole, fumaric acid, malic acid, hydroquinone, dodecanethiol, succinic anhydride or adipic acid;
(C) about 0% to about 15% by weight of a water soluble or water dispersible alcohol having 1 to 6 carbon atoms; and
(D) about 2% to about 33% by weight of water;
wherein the composition has a pH of about 3 to about 5 and the aqueous thermoplastic or thermosetting printing ink is compatible with water at said pH.

WO 96/07670 discloses a compound of the formula: 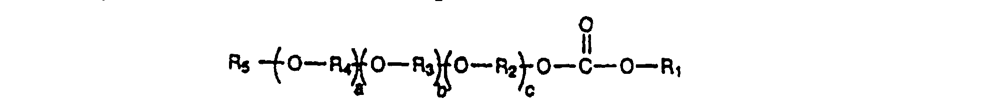 wherein R₁ is selected from an N-benzotriazole group, an N-2-pyrrolidinone group, or an 2-oxypyrimidine group; R₂, R₃ and R₄ are independently selected alkylene groups of about 2 to about 3 carbon atoms and may be the same or different; R₅ is selected from hydrogen, methyl, a carbonyloxy-N-benzotriazole group, a carbonyloxy-N-2-pyrrolidinone group, and a carbonyl-2-oxypyrimidine group; a is an integer from 1 to 1000 and each of b and c is an integer from 0 to 1000, where a + b + c is an integer from 3 to 1000.

US-4464276 discloses polyoxyalkylene polyamine complexes of the formula: 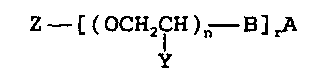 wherein A is 5-aminotriazole or benzotriazole; Y is hydrogen, or a lower alkyl group having 1 to 5 carbon atoms; Z is a hydrocarbon radical having 2-10 carbon atoms forming from 1 to 4 external ether linkages; n is 1 to 15 and r is 1 to 4; B is NH₂ or ureido except that when Z is ureidoalkyl and B is ureido, r can only be 1.

GB-1514359 discloses compounds having the formula: 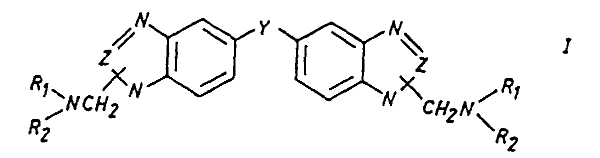 wherein R₁ and R₂ are the same or different and each is hydrogen; an alky group having from 1 to 20 carbon atoms; an alkenyl group having from 2 to 20 carbon atoms; a group of formula

R₃O [(alkylene)O]ₓ(alkylene)―

wherein R₃ is hydrogen or an alkyl residue having from 1 to 20 carbon atoms, alkylene is a straight or branched chain alkylene group having 2 or 3 carbon atoms and x is 0, 1, 2, 3, or 4; a cyanoalkyl group having from 2 to 5 carbon atoms; an aryl group having from 6 to 10 carbon atoms; an aralkyl group having from 7 to 9 carbon atoms; or R₁ and R₂ together may form, with the nitrogen atom, a heterocyclic group; Y is a straight or branched alkylene group having from 1 to 6 carbon atoms, a 1,1-cycloalkylene group having from 5 to 12 carbon atoms, a carbonyl group, a sulphuryl group, an oxygen atom, or a sulphur atom; and Z is N or CR₄ wherein R₄ is hydrogen or a hydrocarbyl residue having from 1 to 8 carbon atoms.

US-5441563 generally describes a printing ink composition comprising a resin, a solvent and an inhibitor; the inhibitor being a compound having the general formula 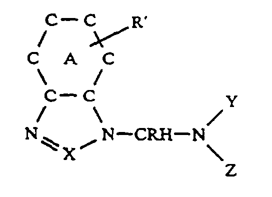 wherein the A ring is benzenoid, naphthenoid or saturated cycloaliphatic, the A ring being unsubstituted or substituted with R' which is an alkyl group of 1 to 4 carbon atoms; R is a hydrogen atom or methyl radical; X is a nitrogen atom or the 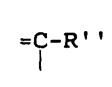 group, wherein R" is a hydrogen atom or an alkyl group of 1 to 4 carbon atoms; and Y and Z are organic moieties or when taken together with the nitrogen to which they are attached form an organic ring structure; the inhibitor having a 24 hour room temperature isopropyl alcohol solubility of less than 5% by weight.

GB-1511593 discloses a compound having the formula 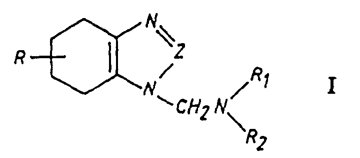 wherein R is hydrogen or methyl; R₁ and R₂ are the same or different and each is a straight or branched alkyl group having from 1 to 20 carbon atoms; an alkenyl group having from 2 to 20 carbon atoms; a residue of formula

R₃O[(CₚH₂ₚ)O]ₓ(CₚH₂ₚ)―

wherein R₃ is hydrogen or an alkyl residue having from 1 to 20 carbon atoms, p is 2 or 3 and x is 0, 1, 2, 3 or 4; a cyanoalkyl group having from 2 to 5 carbon atoms; an aryl group having from 6 to 10 carbon atoms; a cycloalkyl group having from 5 to 12 carbon atoms; or an aralkyl group having from 7 to 9 carbon atoms; or R₁ and R₂ may form, together with the nitrogen atom to which they are attached, the atoms required to complete a heterocyclic ring containing a nitrogen atom and optionally other heteroatoms; or R₁ has its previous significance and R₂ is hydrogen, a heterocyclic residue or a residue having the formula 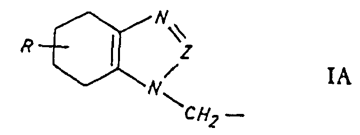 and Z is N or CR₄ wherein R₄ is hydrogen or a hydrocarbyl residue having from 1 to 8 carbon atoms.

GB-1466558 discloses a compound having the formula: 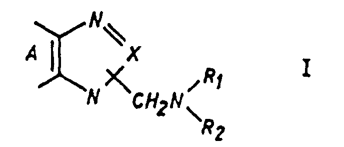 wherein X is N or CR wherein R is hydrogen or an alkyl radical having from 1 to 4 carbon atoms; A is a benzene or naphthalene radical, each unsubstituted or substituted with one or more alkyl radicals having from 1 to 12 carbon atoms or aralkyl radicals having from 7 to 9 carbon atoms; R₁ and R₂ are the same or different and each is a radical of formula R₃O[(alkylene)O]ₓ(alkylene)- wherein R₃ is hydrogen or an alkyl radical having from 1 to 20 carbon atoms, "alkylene" is a straight- or branched chain alkylene radical having 2 or 3 carbon atoms and x is 0, 1, 2, 3 or 4; an alkenyl radical having from 2 to 20 carbon atoms or a cyanoalkyl radical having from 2 to 5 carbon atoms; or R₁ has its previous significance and R₂ is hydrogen, an alkyl radical having from 1 to 20 carbon atoms, an aryl radical having from 6 to 10 carbon atoms, a cycloalkyl radical having from 5 to 12 carbon atoms, an aralkyl radical having from 7 to 9 carbon atoms, a heterocyclic radical or a radical of formula: 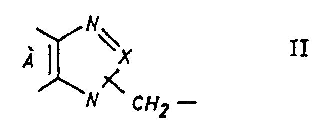 wherein A and X have their previous significance.

As the triazole-based foam-expansion inhibitors, established in the patent literature to date are not soluble in water unless alcohols or other suitably water-miscible organic co-solvents are also present, there continues to exist a need in the art for a water-soluble triazole-based inhibitor which does not require such co-solvents, in order to reduce VOC emissions during the printing and drying process.

In the present invention, by using primary or secondary mono- or diamines in which at least one of the substituents is a polyethylene oxide (PEO) oligomer of sufficient molecular weight, or a polypropylene oxide (PPO)/PEO/PPO triblock oligomer with a sufficiently high PEO/PPO ratio, the resulting aminomethyltriazole will be completely soluble in water without the need for alcohol or other water-miscible organic co-solvents. The PPO/PEO/PPO triblock oligomer has the general formula PPOₓPEO_{y}PPO_{z}, where x, y and z are positive integers.

The number of PEO repeat units required to confer water solubility to the molecule is related to the number of aminomethyltriazole moieties attached to the molecule. The triazole derivatives of the present invention will be soluble in water if the ratio of polyethylene oxide monomer moieties to triazole moieties is at least six and preferably at least eight. The chemical embossing inhibitors embodied in this invention have the advantage that they are inherently soluble in water and can be completely dissolved in aqueous ink formulations without the necessity of added alcohols or other water-soluble co-solvents, or surfactants. However, such co-solvents or surfactants can be added without destroying the present invention.

Accordingly, one subject matter of the present invention is a water soluble triazole derivative having the formula

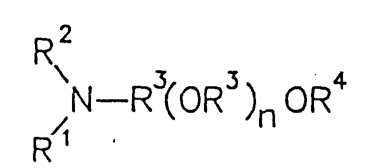

Where:
- R¹=: 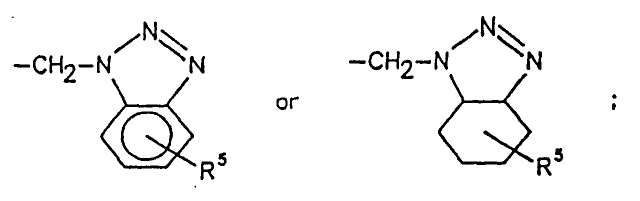
- R²=: -(C₁-C₄) alkyl. -(CH₂CH₂O)ₘCH₃, -(CH₂CH₂O)ₘCH₂CH₃, or -R¹ ;
- R³ =: -CH₂CH₂-. -CH(R⁶)CH₂-, or -CH₂CH(R⁶)- ;
- R⁴ =: -(C₁- C₄) alkyl, or 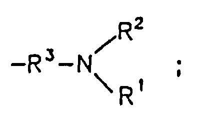
- R⁵ =: -H or -(C₁-C₄) alkyl ;
- R⁶ =: -(C₁-C₄) alkyl ;
n = 5-45;
m = 1-6; and
each of R¹, R², R³, R⁵, and R⁶ may be the same or different.

Another subject matter of the present invention is a printing ink composition for the production of textured foamed surface, which composition comprises a thermoplastic resin, water, and as inhibitor of the formula 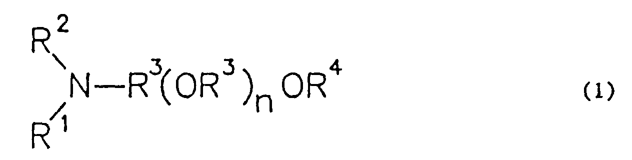 Where:
- R¹ =: 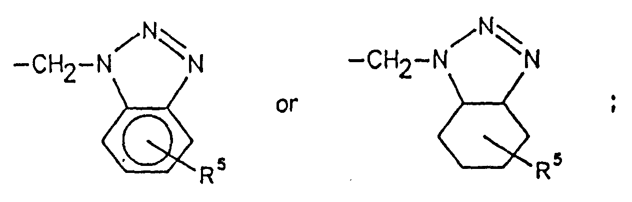
- R²=: - (C₁-C₄) alkyl, -(CH₂CH₂O)ₘCH₃, -(CH₂CH₂O)ₘCH₂CH₃, or -R¹ ;
- R³=: -CH₂CH₂-, -CH(R⁶)CH₂-, or -CH₂CH(R⁶)- ;
- R⁴=: -(C₁-C₄) alkyl, or 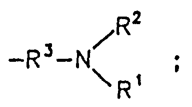
- R⁵=: -H or -(C₁-C₄) alkyl ;
- R⁶ =: -(C₁-C₄) alkyl ;
n = 3-45 ;
m = 1-6 and

Each of R¹, R², R³, R⁵, and R⁶ may be the same or different. R³ is preferably -CH₂CH₂-, -CH(CH₃)CH₂-, or -CH₂CH(CH₃)-. R⁵ is preferably -H or -CH₃.

When R⁴ is -R³N(R¹)alkyl; n is preferably 10 to 25. When R⁴ is -R³N(R¹)₂, n is preferably 20 to 45, more preferably 20 to 35, and most preferably 20 to 30.

When R² is R¹ and R⁴ is -(C₁-C₄)alkyl, n is preferably 10 to 30, and more preferably 15 to 25. When R² is not R¹ and R⁴ is -(C₁-C₄) alkyl, n is preferably 5 to 25, and more preferably 7 to 25.

The water soluble triazole derivatives which have a ratio of polyethylene oxide monomer moieties to triazole moieties of at least six, and preferably a ratio of polyethylene oxide monomer moieties to triazole moieties of at least eight can be used as embossing inhibitors and corrosion inhibitors.

A still further subject matter of the invention is a method of embossing a heat-foamable resinous material by applying the printing ink composition of the present invention to selected areas of the surface of a heat-foamable resinous material, which material contains a blowing agent, and subsequently heating the material to at least the decomposition temperature of the blowing agent.

Structures for some representative examples of the water soluble triazole derivatives of the present invention are shown in the following Tables I and II in which the substituents of Formula (1) are identified; R¹ is identified as aromatic to indicate the first moiety set forth for R¹, supra, or cyclohexyl to indicate the second moiety set forth for R¹, supra. R² is sometimes identified in the same manner. The parenthetical numbers following the moieties of R³ indicate the average number of the OR³ moieties in the compound. The average number of polyethylene oxide monomer moieties and polypropylene oxide monomer moieties is one greater than n.

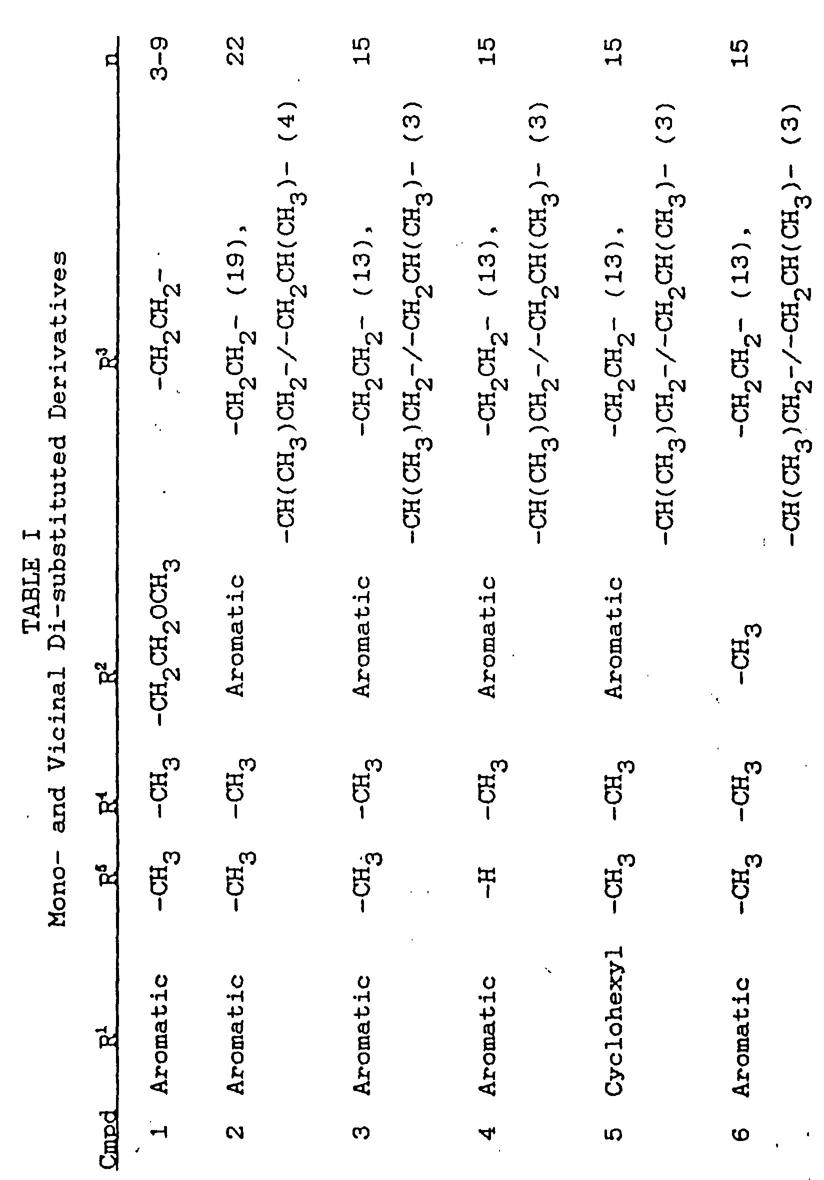

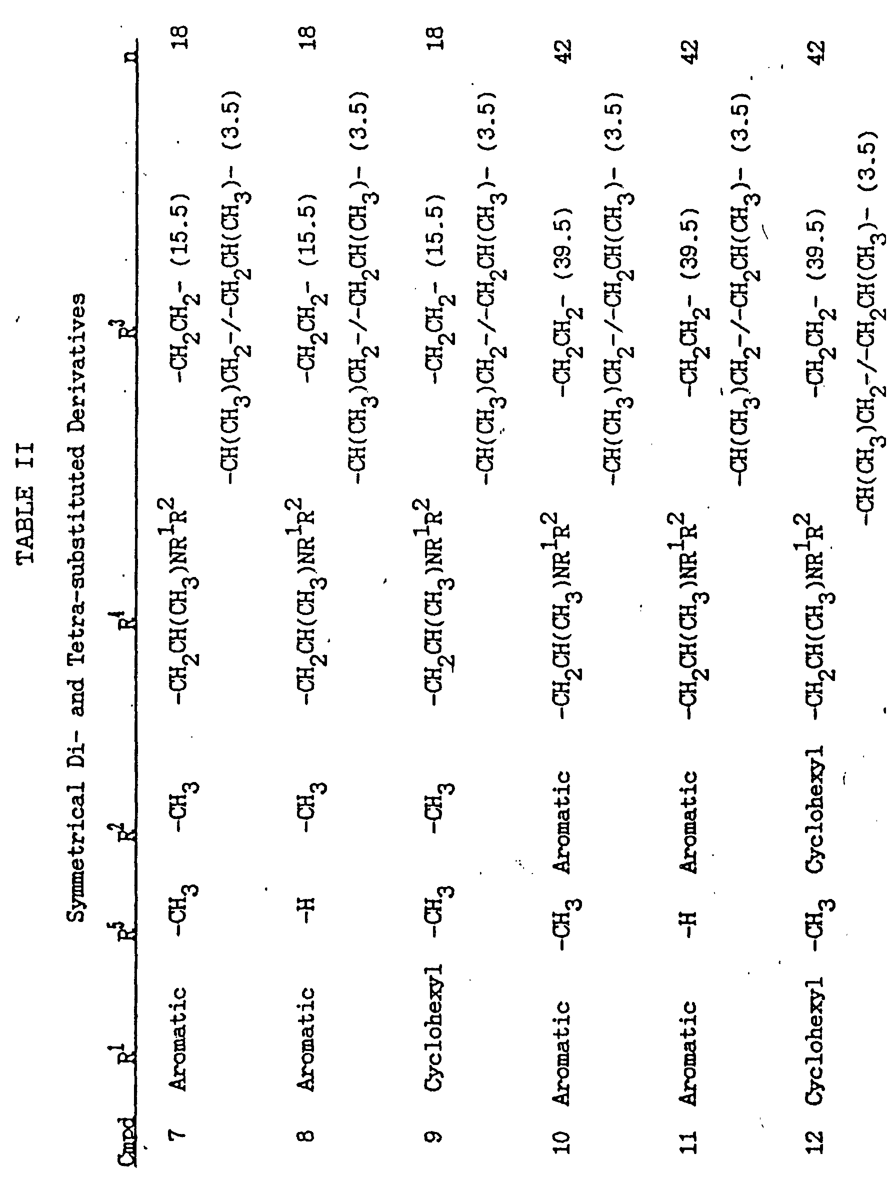

For acceptable processing, it is advantageous to use 5 to 25 parts by weight of the polyalkyleneoxide-derivatized aminomethyltriazoles in the aqueous printing ink composition. Those skilled in the art will recognize that a very wide range of printing ink compositions exist with varying combinations of solubilized and/or dispersible binders, pigments, and rheology-control additives. The pigments are optional, since it may be desirable to use a colorless, inhibitor containing printing ink. The water-soluble triazoles of the present invention are potentially useful in many other aqueous ink formulations not specifically outlined in the Examples as to their exact composition.

Those skilled in the art will also recognize that varying amounts of water will be required to adjust the viscosity of the ink composition to a range suitable for typical rotogravure printing. Other methods of printing the ink composition onto the foamable plastic surface, such as screen printing, relief printing, or planographic printing, may also be used with these ink compositions.

Although this invention is primarily concerned with polyvinylchloride-based plastisol compositions thermally blown with azodicarbonamide as the printing substrate, there likewise exists a wide range of thermoplastic resins which can be thermally foamed with azodicarbonamide and thus are potential substrates for aqueous inhibitor printing ink compositions of the type claimed. Such other compositions include polyvinylacetate, copolymers of vinyl chloride and vinyl acetate, polyacrylate, polymethacrylate, polyethylene, polystyrene, butadiene/styrene copolymers, butadiene/acrylonitrile copolymers, and natural or synthetic rubbers.

The specific combinations of PVC, other thermoplastic resins, filler, stabilizers, liquid plasticizer and chemical blowing agent that make up a typical foamable plastisol substrate vary widely within certain limits and those skilled in the art can reasonably anticipate systems which would be encompassed by the scope of this invention.

The invention is illustrated by the following examples related to synthesis of the water-soluble triazole derivatives, preparation of the aqueous printing ink formulations and demonstration of the chemical embossing behavior of the claimed compounds. Unless otherwise stated, all amounts and percentages given in the Examples are on a weight basis.

### EXAMPLE 1

### Synthesis of Compound 1

### 1-N-[(2-Methoxyethylmethoxypolyethyleneoxy)aminomethyl]tolyltriazole

Sixteen and one-half parts of commercial tolyltriazole (TT100, an isomer mixture from PMC Specialties) and 50.4 parts of the PEO-substituted secondary amine (laboratory prepared) were combined in 150 parts methanol and cooled to zero degrees Centigrade. While holding the reaction mixture at this temperature, 10.1 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 67.1 parts (quantitative yield) of a clear, mobile reddish oil which was identified by standard spectroscopic techniques as the expected Compound 1, 1-N-[(2-methoxyethylmethoxypolyethyleneoxy)aminomethyl]tolyltriazole. The compound was found to be completely miscible with water in all proportions.

### Example 2

### Synthesis of Compound 2

### N,N-Bis(1-N-Tolytriazoylmethyl)polyethylene-Co-Polypropyleneoxyamine

Twenty-six and seven-tenths parts of commercial tolyltriazole and 100.0 parts of the PEO/PPO-substituted primary amine (JEFFAMINE M1000 from Texaco Chemical Co.) were combined in 150 parts methanol and cooled to zero degrees Centigrade. While holding the reaction mixture at this temperature, 16.3 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 128.7 parts (quantitative yield) of a clear, mobile reddish oil which was identified by standard spectroscopic techniques as the expected Compound 2, N,N-bis(1-N-tolytriazoylmethyl)polyethylene-co-polypropyleneoxyamine. The compound was found to be completely miscible with water in all proportions.

### Example 3

### Synthesis of Compound 3

### N,N-Bis(1-N-Tolytriazoylmethyl)polyethylene-Co-Polypropyleneoxyamine

Thirty-seven and three-tenths parts of commercial tolyltriazole and 100.0 parts of the PEO/PPO-substituted primary amine (JEFFAMINE M715 from Texaco Chemical Co.) were combined in 150 parts methanol and cooled to zero degrees Centigrade. While holding the reaction mixture at this temperature, 22.7 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 163.9 parts (quantitative yield) of a clear, mobile reddish oil which was identified by standard spectroscopic techniques as the expected Compound 3. This compound differed from Compound 2 only in the number of repeat units in the PEO/PPO chain and was also found to be completely miscible with water in all proportions.

### Example 4

### Synthesis of Compound 4

### N,N-Bis(1-N-Benzotriazoylmethyl)polyethylene-Co-Polypropyleneoxyamine

Twenty-three and eight tenths parts of commercial benzotriazole and 71.5 parts of the PEO/PPO-substituted primary amine (JEFFAMINE M715 from Texaco Chemical Co.) were combined in 100 parts of methanol and cooled to zero degrees Centigrade. While holding the reaction mixture at this temperature, 16.2 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 97.0 parts (99.3% yield) of a clear, mobile oil, slightly yellow in color, which was identified by standard spectroscopic techniques as the expected Compound 4, N,N-bis(1-N-benzotriazoylmethyl)polyethylene-co-polypropyleneoxyamine. The compound was found to be completely miscible with water in all proportions.

### Example 5

### Synthesis of Compound 5

### N,N-Bis(1-N-Methylcyclohexyltriazoylmethyl)polyethylene-Co-Polypropylene-Oxyamine

Forty and four-tenths parts of hydrogenated tolyltriazole (Cobratec 911 from PMC Specialties) and 103.8 parts of the PEO/PPO-substituted primary amine (JEFFAMINE M715 from Texaco Chemical Co.) were combined in 150 parts of methanol and cooled to zero degrees Centigrade. While holding the reaction mixture at this temperature, 23.5 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 146.8 parts (quantitative yield) of a clear, mobile oil, slightly yellow in color, which was identified by standard spectroscopic techniques as the expected Compound 5, N,N-bis(1-N-methylcyclohexyltriazoylmethyl)polyethylene-co-polypropylene-oxyamine. The compound was found to be completely miscible with water in all proportions.

### Example 6

### Synthesis of Compound 10

### N,N,N',N'-Tetra(1-N-Tolyltriazoylmethyl)polyethylene-Co-Polypropylene-Oxydiamine

Twenty-six and seven-tenths parts of commercial tolyltriazole and 100.0 parts of the PEO/PPO-substituted primary diamine (JEFFAMINE ED-2001 from Texaco Chemical Co.) were combined in 150 parts of methanol and cooled to zero degrees Centigrade. While holding the reaction mixture at this temperature, 16.3 parts of commercial 37% aqueous formaldehyde solution was added slowly over several hours with continual agitation. The reaction mixture was allowed to warm to ambient temperature and worked up after 18 hours by removing the solvent under moderate heat/vacuum. The resulting oil was then vacuum stripped at higher temperature to remove any residual water, unreacted formaldehyde or other volatiles. The final product was 128.6 parts (99.6% yield) of a clear, mobile oil, slightly yellow in color, which was identified by standard spectroscopic techniques as the expected Compound 10, N,N,N',N'-tetra(1-N-tolyltriazoylmethyl)polyethylene-co-polypropylene-Oxydiamine. The compound was found to be completely miscible with water in all proportions.

### EXAMPLES 7 to 10

### Preparation and Testing of Inks (10% Concentration)

Four inks were made using the compounds from Examples 1, 2, 3 and 6. These compounds were added directly to Sicpa's anionic water-based ink extender 694550 at a concentration of 10% active inhibitor. These compounds readily solubilized into the ink extender without any adverse reactions. Two controls were evaluated at the same time (i.e., 10% Benzotriazole and 8% Trimellitic anhydride) in a solvent-base extender.

All five inks were printed into 0.23 mm of an expandable plastisol coated on flooring felt and on 0.18 mm of an expandable plastisol coated onto a saturated glass mat. The plastisol formulation coated on the flooring felt was 100 parts by weight PVC resin, 50 parts plasticizer, 30 parts limestone filler, 7.0 parts titanium dioxide pigment, 3.0 parts mineral spirits viscosity modifier, 2.1 parts stabilizers, 2.0 parts azodicarbonamide blowing agent and 0.6 parts zinc oxide blowing agent activator. The printing was done on a flat-bed gravure proof press using a 100 line screen step-wedge engraved plate. The steps ranged from a deep shadow tone to a shallow highlight tone.

The printed samples were coated with 0.25 mm of a clear plastisol wearlayer, and fused and expanded in a Werner Mathis oven. The clear wearlayer was 100 parts by weight PVC resin, 40 parts plasticizer, 4.0 parts stabilizers and 4.0 parts mineral spirits. The felt backed structure was heated for 1.3 ± 0.1 minutes at an air temperature of 201° ± 1° C to a blow ratio of about 2.8 to 1.

The plastisol formulation coated on the glass mat was 100 parts by weight PVC resin, 55 parts plasticizer, 30 parts limestone filler, 5.0 parts titanium dioxide pigment, 3.0 parts mineral spirits viscosity modifier, 2.0 parts azodicarbonamide blowing agent and 0.5 parts zinc oxide blowing agent activator. The printing was done on a flat-bed gravure proof press using a 40 raster screen step-wedge engraved plate. The steps ranged from a deep shadow tone to a shallow highlight tone.

The printed samples were coated with 0.25 mm of a clear plastisol wearlayer, and fused and expanded in a Werner Mathis oven. The clear wearlayer was 100 parts by weight PVC resin, 50 parts plasticizer and 2.0 parts stabilizers. The glass backed structure was heated for 1.9 ± 0.1 minutes at an air temperature of 185° ± 2° C to a blow ratio of about 2.0 to 1.

The thickness of the printed areas (i.e., restricted area) was measured in mils and compared to the thickness of the unprinted expanded surrounding areas. This difference is reported as the depth of chemical embossing and is shown in Table III.

**TABLE III**

| INHIBITOR | WEIGHT PERCENT OF COMPOUND IN INK | DEPTH OF EMBOSSING FOR FELT STRUCTURE in mm | DEPTH OF EMBOSSING FOR GLASS STRUCTURE in mm |
|---|---|---|---|
| Example 7 (Compound 1) | 10% | 0.145 | 0.119 |
| Example 8 (Compound 2) | 10% | 0.112 | 0.074 |
| Example 9 (Compound 3) | 10% | 0.140 | 0.099 |
| Example 10 (Compound 10) | 10% | 0.076 | 0.081 |
| BTA | 10% | 0.300 | 0.089 |
| TMA | 8% | 0.218 | 0.150 |

### EXAMPLES 11 to 13

### Preparation and Testing of Inks (15% Concentration)

The following three inks were made using compounds from Examples 3, 4 and 5. They were mixed with Sicpa's anionic water-based ink extender 694556 at a concentration of 15% by weight, without any problems. A 10% Benzotriazole solvent-based ink control was used for this evaluation. These inks were printed on the same felt backed and glass backed structures used in Table III and evaluated by the same method for the depth of chemical embossing (see Table IV).

**TABLE IV**

| INHIBITOR | WEIGHT PERCENT OF COMPOUND IN INK | DEPTH OF EMBOSSING FOR FELT STRUCTURE in mm | DEPTH OF EMBOSSING FOR GLASS STRUCTURE in mm |
|---|---|---|---|
| Example 11 (Compound 3) | 15% | 0.173 | 0.124 |
| Example 12 (Compound 4) | 15% | 0.218 | 0.122 |
| Example 13 (Compound 5) | 15% | 0.226 | 0.109 |
| BTA | 10% | 0.297 | 0.099 |

### EXAMPLES 14 to 16

### Preparation and Testing of Compound 3 Inks (10%, 15% & 20% Concentration)

Example 3 (Compound 3) was evaluated at three concentrations to see if the depth of chemical embossing would improve with higher concentrations. This compound was added to Sicpa's anionic water-based ink extender 694556 at three concentrations (i.e., 10%, 15% and 20%). A 10% Benzotriazole solvent-based ink control was used on the same felt backed structure coated with 9 mils of expandable plastisol. The same method used previously was used to evaluate the chemical embossing depth (see Table V).

**TABLE V**

| INHIBITOR | WEIGHT PERCENT OF COMPOUND IN INK | DEPTH OF EMBOSSING FOR FELT STRUCTURE in mm |
|---|---|---|
| Example 14 (Compound 3) | 10% | 0.145 |
| Example 15 (Compound 3) | 15% | 0.206 |
| Example 16 (Compound 3) | 20% | 0.254 |
| BTA | 10% | 0.333 |

## Claims

1. A water soluble triazole derivative having the formula 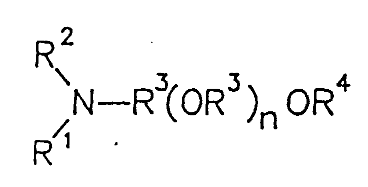 Where:
R¹= 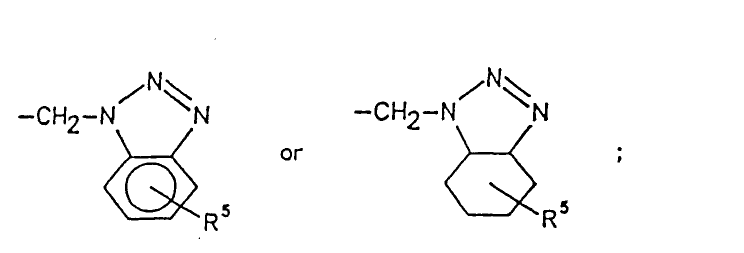
R²= -(C₁-C₄) alkyl, -(CH₂CH₂O)ₘCH₃, -(CH₂CH₂O)ₘCH₂CH₃, or -R¹ ;
R³= -CH₂CH₂-, -CH(R⁶)CH₂-, or -CH₂CH(R⁶)- ;
R⁴ = -(C₁ - C₄) alkyl, or 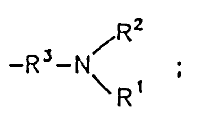
R⁵= -H or -(C₁-C₄) alkyl ;
R⁶= -(C₁-C₄) alkyl ;
n = 5-45;
m = 1-6; and
Each of R¹, R², R³, R⁵, and R⁶ may be the same or different.

2. The compound of claim 1 wherein the R¹ is aromatic.

3. The compound of claim 1 wherein the compound is mono-triazole substituted.

4. The compound of claim 1 wherein the compound is di-triazole substituted.

5. The compound of claim 1 wherein n = 10 to 25 and R⁴ is a mono-triazole substituted amino moiety.

6. The compound of claim 1 wherein n = 20 to 45 and R⁴ is a di-triazole substituted amino moiety.

7. The compound of claim 1 wherein n = 5 to 25, R⁴ is an alkyl moiety, and R² is other than a triazole moiety.

8. A printing ink composition comprising a thermoplastic resin, water and an inhibitor of the formula 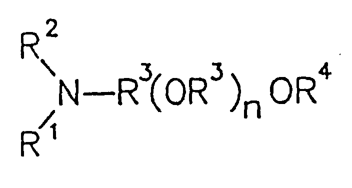 Where:
R¹= 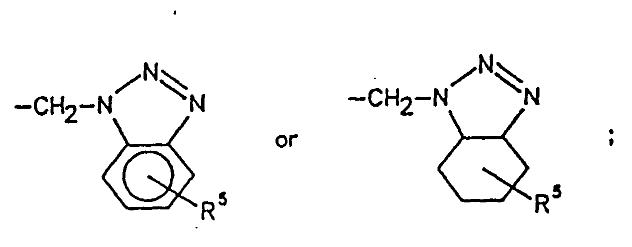
R²= -(C₁-C₄) alkyl, -(CH₂CH₂O)ₘCH₃,-(CH₂CH₂O)ₘCH₂CH₃, or -R¹ ;
R³ = -CH₂CH₂-, -CH(R⁶)CH₂-, or -CH₂CH(R⁶)-;
R⁴= - (C₁- C₄) alkyl, or 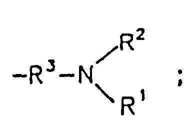
R⁵ = -H or -(C₁-C₄) alkyl ;
R⁶= -(C₁-C₄) alkyl ;
n = 3 -45 ;
m = 1-6 ; and
Each of R¹, R², R³, R⁵, and R⁶ may be the same or different.

9. A method of embossing a heat-foamable, resinous material comprising applying the printing ink composition of claim 8 to selected areas of the surface of the heat-foamable material, which heat-foamable material contains a blowing agent, and heating the heat-foamable material to at least the decomposition temperature of the blowing agent.

10. The method of claim 9, wherein the heat-foamable material is selected from the group consisting of polyvinylchloride, polyvinylacetate, copolymers of vinyl chloride and vinyl acetate, polyacrylate, polymethacrylate, polyethylene, polystyrene, butadiene/styrene copolymers, butadiene/acrylonitrile copolymers, and natural or synthetic rubbers.

11. The structure obtained according to claim 9.

## Patentansprüche

1. Wasserlösliches Triazolderivat der Formel 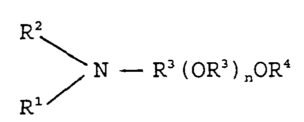 worin
R¹ 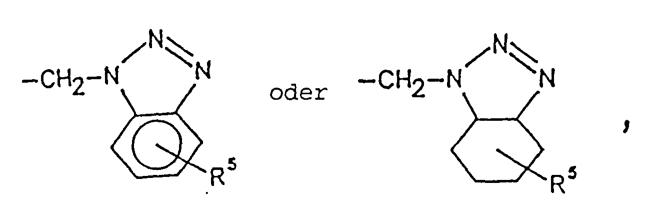
R² = - (C₁-C₄) -Alkyl, - (CH₂CH₂O)ₘCH₃, - (CH₂CH₂O)ₘCH₂CH₃ oder -R¹,
R³ = -CH₂CH₂-, -CH(R⁶)CH₂- oder -CH₂CH(R⁶)-,
R⁴ = 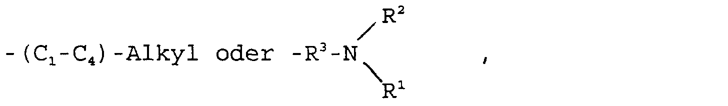
R⁵ = -H oder - (C₁-C₄)-Alkyl,
R⁶ = -(C₁-C₄)-Alkyl,
n = 5 - 45,
m = 1 - 6 und
R¹, R², R³, R⁵ und R⁶ jeweils dieselben oder unterschiedliche Bedeutungen haben.

2. Verbindung nach Anspruch 1, worin R¹ aromatisch ist.

3. Verbindung nach Anspruch 1, worin die Verbindung monotriazolsubstituiert ist.

4. Verbindung nach Anspruch 1, worin die Verbindung ditriazolsubstituiert ist.

5. Verbindung nach Anspruch 1, worin n 10 bis 25 bedeutet und R⁴ ein monotriazolsubstituiertes Aminofragment ist.

6. Verbindung nach Anspruch 1, worin n 20 bis 45 bedeutet und R⁴ ein ditriazolsubstituiertes Aminofragment ist

7. Verbindung nach Anspruch 1, worin n 5 bis 25 bedeutet, R⁴ ein Alkylfragment und R² kein Triazolfragment sind.

8. Druckfarbengemisch, das ein thermoplastisches Harz, Wasser und einen Inhibitor der Formel 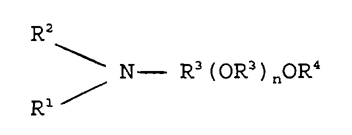 worin
R¹ = 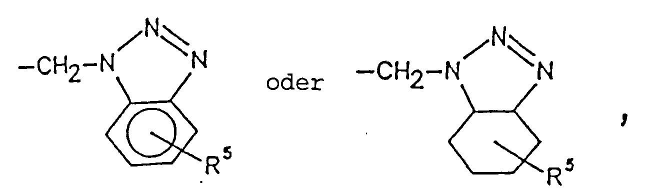
R² = - (C₁-C₄)-Alkyl, - (CH₂CH₂O)ₘCH₃, - (CH₂CH₂O)ₘCH₂CH₃ oder -R¹,
R³ = -CH₂CH₂-, -CH(R⁶)CH₂- oder -CH₂CH(R⁶)-,
R⁴ = - (C₁-C₄) -Alkyl oder 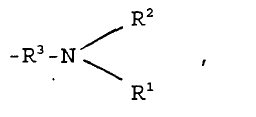
R⁵ = -H oder - (C₁-C₄)-Alkyl,
R⁶ = -(C₁-C₄)-Alkyl,
n = 3 bis 45
m = 1 bis 6 und
R¹, R², R³, R⁵ und R⁶ dieselben oder unterschiedliche Bedeutungen haben, umfasst.

9. Verfahren zum Prägen von warmschäumbarem Harzmaterial, welches das Aufbringen des Druckfarbengemisches gemäß Anspruch 8 auf ausgewählte Bereiche der Oberfläche des warmschäumbaren Materials, das ein Blähmittel enthält, und die Erwärmung des warmschäumbaren Materials zumindest auf die Zersetzungstemperatur des Blähmittels umfasst.

10. Verfahren nach Anspruch 9, bei dem das warmschäumbare Material ausgewählt wird aus der Gruppe, bestehend aus Polyvinylchlorid, Polyvinylacetat, Vinylchlorid-Vinylacetet-Copolymeren, Polyacrylat, Polymethacrylat, Polyethylen, Polystyrol, Butadien-Styrol-Copolymeren, Butadien-Acrylnitril-Copolymeren und Natur- oder Synthesekautschuken.

11. Gebilde, erhalten gemäß Anspruch 9.

## Revendications

1. Dérivé de triazole hydrosoluble répondant à la formule 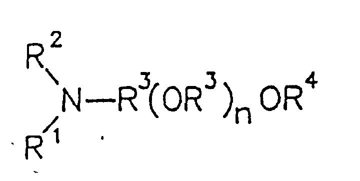 dans laquelle
R¹ représente 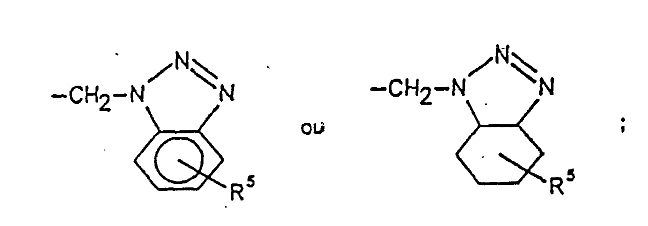
R² représente un groupe alkyle en C₁-C₄, un groupe -(CH₂CH₂O)ₘCH₃, un groupe -(CH₂CH₂O)ₘCH₂CH₃ ou R¹;
R³ représente un groupe -CH₂CH₂-, un groupe -CH(R⁶)CH₂ ou un groupe -CH₂CH(R⁶)-,
R⁴ représente un groupe alkyle en C₁-C₄ ou un groupe 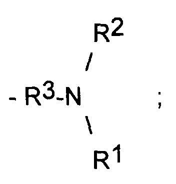
R⁵ représente un atome d'hydrogène ou un groupe alkyle en C₁-C₄;
R⁶ représente un groupe alkyle en C₁-C₄;
n = 5-45;
m= 1 - 6; et
chacun des radicaux R¹, R², R³, R⁵ et R⁶ peuvent être identiques ou différents.

2. Composé selon la revendication 1, dans lequel R¹ représente un radical aromatique.

3. Composé selon la revendication 1, dans lequel le composé est substitué par un monotriazole.

4. Composé selon la revendication 1, dans lequel le composé est substitué par un ditriazole.

5. Composé selon la revendication 1, dans lequel n = 10 à 25 et R⁴ représente une fraction amino substituée par un monotriazole.

6. Composé selon la revendication 1, dans lequel n = 20 à 45 et R⁴ représente une fraction amino substituée par un ditriazole.

7. Composé selon la revendication 1, dans lequel n = 5 à 25, R⁴ représente une fraction alkyle et R² représente autre chose qu'une fraction triazole.

8. Composition d'encre d'impression comprenant une résine thermoplastique, de l'eau et un inhibiteur répondant à la formule 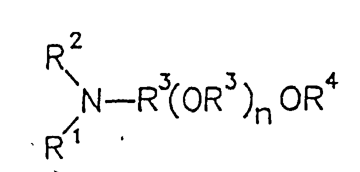 dans laquelle
R¹ représente 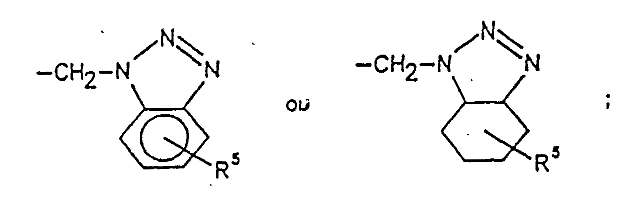
R² représente un groupe alkyle en C₁-C₄, un groupe -(CH₂CH₂O)ₘCH₃, un groupe -(CH₂CH₂O)ₘCH₂CH₃ ou R¹;
R³ représente un groupe -CH₂CH₂-, un groupe -CH(R⁶)CH₂ ou un groupe -CH₂CH(R⁶)-,
R⁴ représente un groupe alkyle en C₁-C₄ ou un groupe 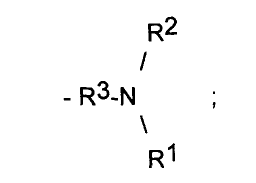
R⁵ représente un atome d'hydrogène ou un groupe alkyle en C₁-C₄;
R⁶ représente un groupe alkyle en C₁-C₄;
n = 3 - 45;
m = 1 - 6; et
chacun des radicaux R¹, R², R³, R⁵ et R⁶ peuvent être identiques ou différents.

9. Procédé de gaufrage d'une matière résineuse apte à être transformée en mousse sous l'influence de la chaleur, comprenant le fait d'appliquer la composition d'encre d'impression selon la revendication 8 sur des zones sélectionnées de la matière apte à être transformée en mousse sous l'influence de la chaleur, ladite matière apte à être transformée en mousse sous l'influence de la chaleur contenant un agent moussant, et de chauffer la matière apte à être transformée en mousse sous l'influence de la chaleur jusqu'à au moins la température de décomposition de l'agent moussant.

10. Procédé selon la revendication 9, dans lequel la matière apte à être transformée en mousse sous l'influence de la chaleur est choisie parmi le groupe constitué par le chlorure de polyvinyle, l'acétate de polyvinyle, des copolymères de chlorure de vinyle et d'acétate de vinyle, du polyacrylate, du polyméthacrylate, du polyéthylène, du polystyrène, des copolymères de butadiène/styrène, des copolymères de butadiène/acrylonitrile et des caoutchoucs naturels ou synthétiques.

11. Structure obtenue conformément à la revendication 9.
